# EUROPEAN PATENT APPLICATION

(11) **EP 4 183 867 A1**
(43) Date of publication of application: **24.05.2023**
(21) Application number: 21843534.5
(22) Date of filing: 13.07.2021
(51) Int. Cl.: C12N 5/071, C12M 3/00

(54) **METHOD FOR PRODUCING CARDIOMYOCYTE CELL MASS**

(30) Priority: 14.07.2020 JP 2020120695
(71) Applicant: Kaneka Corporation, Osaka-shi, Osaka 530-8288 (JP)
(72) Inventor: KAWAI, Yoshikazu, Kobe-shi, Hyogo 650-0047 (JP)
(74) Representative: Hoffmann Eitle
(86) International application number: PCT/JP2021/026374
(87) International publication number: WO 2022/014622

(57) **Abstract**

A large amount of cardiomyocyte spheroids is obtained from dissociated cardiomyocytes in a simple manner, in a short time, without using a special culture medium component, and without using a complicatedly-shaped container or a minute container. The problems can be solved by a method for producing cardiomyocyte spheroids, the method including allowing dissociated cardiomyocytes to flow under suspension conditions in a non-annular container to aggregate the cells. In addition, the problems can also be solved by a method for producing cardiomyocyte spheroids, in which the dissociated cardiomyocytes have a history of stabilization culture after thawing.

## Description

### Technical Field

The present invention relates to a method for producing cardiomyocyte spheroids.

### Background Art

Cardiomyocyte transplantation is being proposed as a method for treatment of a serious cardiac disease. Examples of cardiomyocyte transplantation forms are sheets or spheroids. Among these, cardiomyocyte spheroids can be injected directly into the heart using an injection needle, and thus, is considered to provide a high therapeutic effect. Such a transplantation requires a relatively large amount of cardiomyocytes, and hence, there is a demand for a technique to prepare a large amount of cardiomyocytes with a high possibility. As one such technique, a technique for producing cardiomyocytes by inducing the differentiation of a pluripotent stem cell is considered to be promising. When the differentiation of a pluripotent stem cell is induced, the cells are often in the form of an aggregate, and thus a method for purifying usually involves dispersing and dissociation of once. Patent Literature 1 discloses a method for preparing cardiomyocyte spheroids back from the dissociated cardiomyocytes, in which a liquid obtained by suspending cardiomyocytes in culture medium containing a special component is put into a roundbottomed 96-well plate. However, the method is unsuitable as a preparation method of obtaining a required number of cardiomyocyte spheroids for treatment, and the Examples thereof states that 10 days were required from the time when the preparation of cardiomyocyte spheroids starts to the time when the cardiomyocytes start beating.

### Citation List

### Patent Literature

Patent Literature 1: JP5523830B2

### Summary of Invention

### Technical Problem

An object of the present invention is to obtain a large amount of cardiomyocyte spheroids from dissociated cardiomyocytes in a simple manner, in a short time, without using special culture medium component, or a container with a complicated-shape or a minute container.

### Solution to Problem

The present invention described below solved the above-mentioned problems.
(1) A method for producing cardiomyocyte spheroids, comprising allowing dissociated cardiomyocytes to flow under suspension conditions in a container to aggregate the cells, wherein the vertical cross section and horizontal cross section of that portion of the container which is in contact with a cell suspension are each uniform and single-layered.
(2) A method for producing cardiomyocyte spheroids, comprising allowing dissociated cardiomyocytes having a history of stabilization culture to flow under suspension conditions to aggregate the cells.
(3) The method for producing cardiomyocyte spheroids, wherein the stabilization culture is adherent culture.
(4) The method for producing cardiomyocyte spheroids, wherein the period for the stabilization culture is 4 hours or more.
(5) The method for producing cardiomyocyte spheroids, wherein the time taken to produce the cardiomyocyte spheroids is within 216 hours.
(6) The method for producing cardiomyocyte spheroids, wherein the container is substantially flat-bottomed.
(7) The method for producing cardiomyocyte spheroids, wherein the container has a capacity of 0.5 mL or more.
(8) The method for producing cardiomyocyte spheroids, wherein the flow is performed by rotation at 20 rpm or more and less than 200 rpm.
(9) The method for producing cardiomyocyte spheroids, wherein the container is rotated in the range of 10 mm or more.
(10) The method for producing cardiomyocyte spheroids, wherein the time without using culture medium containing the following component(s): insulin, transferrin, selenium, basic fibroblast growth factor (bFGF), epidermal growth factor (EGF), platelet-derived growth factor-BB (PDGF-BB), and/or endothelin-1 (ET-1) is 26 hours or more during the production of the cardiomyocyte spheroids.
(11) The method for producing cardiomyocyte spheroids, wherein the cardiomyocytes are derived from a pluripotent stem cell(s).
(12) Cardiomyocyte spheroids having an average aspect ratio of 0.70 or more.
(13) Cardiomyocyte spheroids, wherein the standard deviation of the size of the cardiomyocyte spheroids is 30% or less of the average in the size of the cardiomyocyte spheroids.
(14) Cardiomyocyte spheroids, wherein the average size of the cardiomyocyte spheroids is 50 µm or more and 2000 µm or less.
(15) Cardiomyocyte spheroids that starts beating within 216 hours from the start of production of the cardiomyocyte spheroids.

The present specification encompasses the disclosure of Japanese Patent Application No. 2020-120695 that serves as a basis for the priority of the present application.

### Advantageous Effects of Invention

The present invention allows to obtain cardiomyocyte spheroids in a large amount, in a simple manner, and in a short time. A contribution to the propagation of cardiomyocyte transplant is expected is expected.

### Brief Description of Drawings

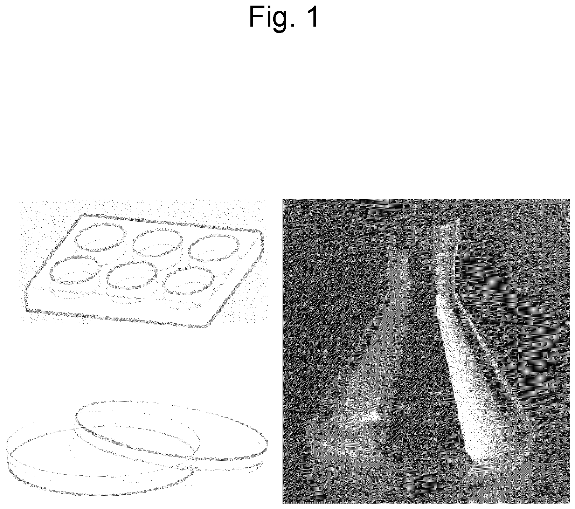

[Figure 1] Figure 1 is an example of a container having a flat bottom and having a portion to be in contact with a cell suspension with its vertical cross section and horizontal cross section are each uniformed and single-layered.

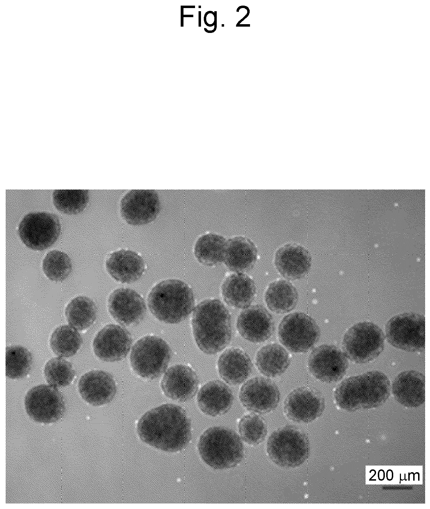

[Figure 2] Figure 2 is a phase-contrast microscopic image in Example 7 of the present invention.

### Description of Embodiments

The present invention is to provide a method for producing cardiomyocyte spheroids, comprising allowing dissociated cardiomyocytes to flow under suspension conditions in a container to aggregate the cells. This allows to obtain cardiomyocyte spheroids in a large amount, in a simple manner, and in a short time, and a contribution to the propagation of cardiomyocyte transplant is expected. As used herein, the term "cardiomyocyte spheroid" refers to a spheroid formed by aggregating a plurality of cardiomyocytes three-dimensionally.

Cardiomyocytes to be used in the present invention is not particularly limited. Cardiomyocytes harvested from an organism may be used directly or after being purified or grown. Alternatively, cardiomyocytes induced from a stem cell or the like may also be used. Cardiomyocytes are preferably obtained by induction of differentiation from a pluripotent stem cell, as below-mentioned. A method of dissociation of cardiomyocytes is also not particularly limited, and a conventionally known method may be used. Dissociated cardiomyocytes are preferably purified cells. A method of purifying cardiomyocytes is not particularly limited, and a conventionally well-known method may be used. Examples of such a method include: using culture medium for pluripotent stem cells with low-nutrients; using relatively high culture temperature; adding a substance that acts specifically on a pluripotent stem cell; and the like. Examples of an indicator of purity of the cardiomyocytes include cardiac troponin T-positivity. The purity of cardiomyocytes that can be used for the present cell is not particularly limited, but preferably exhibits cardiac troponin T-positivity at 20 days after the start of the induction of differentiation. In addition, the ratio of cardiac troponin T-positive cells in the dissociated cells to be used in the present invention is preferably 50% or more in terms of obtaining cardiomyocyte spheroids having high quality, more preferably 80% or more, most preferably 95% or more. The ratio of cardiac troponin T-positive cells can be determined by a conventionally known immunological technique such as a flow cytometric method with fluorescence labeling or an electrochemiluminescent immunoassay (ECLIA). In addition, cardiomyocytes to be used are more preferably ventricular cardiac muscle cells in terms of a high effect of treatment. Examples of a marker for ventricular cardiac muscle cells include MLC2v.

Forming cardiomyocyte spheroids using dissociated cardiomyocytes often provide cardiomyocyte spheroids having favorable sphericity and spheroid diameter with the relatively narrow distribution. Usually, as seen in The Journal of Cell Biology 143 (5): 1341-52, the shape of the cell spheroid during induction of differentiation often exhibit low sphericity, and the wide distribution of the spheroid diameter. Cardiomyocyte spheroids having favorable sphericity and narrow spheroid diameter distribution causes less trouble when injected, and in addition, makes it more possible to obtain a homogeneous therapeutic effect.

The present invention is characterized in that cardiomyocytes and/or cardiomyocyte spheroids are formed under suspension conditions. A suspension condition literally relates to a state where the cells are suspended in a liquid component such as culture medium, and encompasses a state where the cells can be separated from a base material by a small stimulus, even if the cells are sedimented at the bottom of a container. From such a viewpoint, the surface of the base material is preferably not a material adhesive or fixative to the cells. The density of cardiomyocytes in a suspension is not particularly limited, and is preferably 1 × 10³ cells/mL or more, in terms of reducing the cost of a liquid component such as culture medium and increasing the formation of cell spheroids from the cells, more preferably 1 × 10⁴ cells/mL or more, particularly preferably 1 × 10⁵ cells/mL or more, most preferably 2 × 10⁵ cells/mL or more. In addition, the density of cardiomyocytes is preferably 1 × 10⁸ cells/mL or less, in terms of inhibiting cardiomyocytes from being aggregated excessively, still more preferably 1 × 10⁷ cells/mL or less, particularly preferably 1 × 10⁶ cells/mL or less, most preferably 5 × 10⁵ cells/mL or less.

A method for producing cardiomyocyte spheroids according to the present invention is performed in a container. The shape of the container is not particularly limited, and a commercially available versatile structure may be used. Examples of a container having a versatile structure include a non-annular container. A non-annular container is a container that is not annular, and a container having a structure in which the vertical cross section and horizontal cross section of the portion to be in contact with a cell suspension are uniform and single-layered. The vertical direction means a direction vertical to the liquid surface of the cell suspension left to stand, and the horizontal direction means a direction parallel to the liquid surface of the cell suspension left to stand. Using such a non-annular container enhances the easiness of scale-up. Specific examples of a non-annular container include an easily available container with a simple structure in which only wall is an outer wall, such as a 6-well plate, 12-well plate, Schale-shaped container, and flask container. In case where the container is a well plate, "uniform and single-layered" means that the cross section of each well is uniform and single-layered. On the other hand, an annular container is a container having what is called an annular structure, such as, a container of a doughnut type (or a swimming ring type). In addition, a container to be used in the present invention is preferably substantially flat-bottomed. Being "substantially flat-bottomed" means that 60% or more of the area of the bottom plate is planar. In addition, examples of a container that is not flat-bottomed include a round-bottom plate (a U-bottom plate), V-bottom plate, and the like. In addition, a container to be used in the present invention preferably has a capacity of 0.5 mL or more. The capacity is preferably 0.5 mL or more in terms of producing cardiomyocyte spheroids in a large amount, more preferably 6.0 mL or more, still more preferably 15.0 mL or more. The capacity is particularly preferably 100 mL or more in terms of commercial production, preferably 500 mL or more in terms of treating a patient with a relatively small body, and preferably 1000 mL or more in terms of treating a relatively large patient. In addition, the capacity is preferably 70 L or less in terms of producing a cell spheroid with favorable handleability, more preferably 15 L or less, still more preferably 7 L or less, particularly preferably 4 L or less, most preferably 3 L or less. The capacity of the container is preferably a volume recommended by the manufacturer thereof, considering a spatter of liquid during flowing, and the like. In addition, in cases where the container is a well plate, the capacity means a capacity per well. In addition, the container is preferably sterilized, and more preferably disposable. The container is preferably ensured its sterile property during the production of cardiomyocyte spheroids. For example, the container preferably has a structure which is a closed system except for a filter having a small aperture (for example, 0.2 µm or less) to supply and exhaust an air and/or a liquid.

A method for producing cardiomyocyte spheroids according to the present invention is characterized by being performed while the cardiomyocyte spheroids are allowed to flow in the container. For example, the method can be performed as follows: the container containing a suspension containing cardiomyocytes is placed on a stage held substantially horizontally, and the stage is rotated in the horizontal direction. As rotation continues, and after a while, the cells are aggregated with each other to form cell spheroids. The rotation speed is not particularly limited, but is preferably 20 rpm or more because this in terms of inhibiting the cells from being aggregated excessively, more preferably 46 rpm or more, still more preferably 62 rpm or more, particularly preferably 83 rpm or more, most preferably 90 rpm or more. In addition, the rotation speed is preferably less than 200 rpm in terms of yield, more preferably less than 170 rpm, still more preferably less than 140 rpm, particularly preferably less than 120 rpm, most preferably less than 110 rpm. The range for rotating the container is not particularly limited, but is preferably 10 mm or more in terms of increasing the cells to aggregate with each other, is particularly preferably 15 mm or more, most preferably 20 mm or more. In addition, the rotation range is preferably 100 mm or less in terms of reducing cell deaths, is more preferably 80 mm or less, still more preferably 60 mm or less, particularly preferably 45 mm or less, most preferably 30 mm or less.

A cardiomyocyte transplant treatment requires a relatively large amount of cardiomyocytes. In addition, a large amount of cell is required as a raw material in order to obtain cardiomyocytes by induction of differentiation. A pluripotent stem cell such as an induced pluripotent stem cell (iPS cell) or an embryonic stem cell (ES cell) can infinitely grow by its nature, and thus, is suitable as the raw material for cardiomyocytes to be used in the present invention. A nonneoplastic pluripotent stem cell such as a Muse cell is also preferable from a safety viewpoint. In addition, in cases where cardiomyocyte spheroids are used for treatment of a human, cardiomyocytes are preferably derived from a human pluripotent stem cell.

Obtaining cardiomyocytes from a pluripotent stem cell often involves performing a differentiation-inducing step for tens of days. In addition, growing pluripotent stem cells takes several weeks in some cases. The total of the time taken to grow pluripotent stem cells and the time taken to induce myocardial differentiation results in quite a long. For example, if a decision on cardiomyocytes transplant treatment is followed by starting the growth of pluripotent stem cells, several months will be taken until cardiomyocyte spheroids transplant is performed. In addition, it is often difficult to install a large-scale cell production equipment in a facility for a transplant treatment. To avoid such a problem, it is preferable to prepare a large amount of cells or cell spheroids in advance, and freeze-preserve them. A step of freeze-preservation is not particularly limited. For example, the freeze-preservation is preferably performed in one or more steps as follows: during or after the growth of pluripotent stem cells; during the induction or after completion of the induction of myocardial differentiation; after the dissociation of cardiomyocytes; during or after the purification of cardiomyocytes; or after the formation of cardiomyocyte spheroids. In terms of shortening the period of time from the thawing to the use of cardiomyocyte spheroids, the freeze-preservation is performed more preferably in one or more steps after the start of induction of myocardial differentiation, the freeze-preservation is performed still more preferably in one or more steps after the induction of myocardial differentiation, the freeze-preservation is performed particularly preferably in one or more steps during or after the purification of the cardiomyocytes, the freeze-preservation is performed most preferably in one or more steps after the dissociated cardiomyocytes having a preferable purity are obtained. In terms of using cardiomyocyte spheroids for a transplant treatment or the like rapidly, the period of time from the thawing to the formation of the cardiomyocyte spheroids and the start of beating of the cell spheroids is preferably within 216 hours in terms of providing the cell spheroids in short time for cardiomyocytes transplant treatment or the like, more preferably within 132 hours, still more preferably within 108 hours, particularly preferably within 84 hours, most preferably within 60 hours.

In addition, the cells to be frozen and thawed are preferably dissociated, in terms of homogenizing the history of the temperature the cells experience during being frozen and thawed.

The present inventors have discovered that using dissociated cardiomyocytes having a history of stabilization culture to produce cardiomyocyte spheroids allows to obtain cardiomyocyte spheroids having high sphericity and spheroid diameter with narrow distribution, very efficiently. Stabilization culture refers to a culture performed after the thawing of the cells and before the start of production of cardiomyocyte spheroids. An operation to be performed during the stabilization culture is not particularly limited, but it is preferable that the state of the cells is not intendedly changed significantly. For example, it is preferable that induction of differentiation is not performed on purpose. A step of stabilization culture is not particularly limited, and is preferably performed immediately before the start of production of cardiomyocyte spheroids, in terms of obtaining cardiomyocyte spheroids having a favorable shape efficiently. As used here, the expression "efficiently" encompasses a higher possibility of generating no dead cells. In addition, performing the stabilization culture immediately after the last thawing before the production of cardiomyocyte spheroids is also preferable because such a manner makes it more possible to efficiently obtain cardiomyocyte spheroids having a favorable shape. In addition, the stabilization culture is preferably performed using an adhesion method. Cell culture by an adhesion method (adherent culture) means that a culture is performed not under the suspension conditions, but in a state where the cells are immobilized on a base material to some extent. The surface of the base material preferably comprises a material adhesive to the cells. A method of allowing the surface of the base material to contain a material adhesive to the cells can be performed by treatment by a known method using a conventionally known coating agent. In addition, when the stabilization culture is completed, a base material from the surface of which the cells are easily released, or a releasing agent and/or a chelator is/are used. The releasing agent is not limited, and for example, trypsin, collagenase, pronase, hyaluronidase, and elastase, and, commercially available Accutase (registered trademark), Accumax (registered trademark), TrypLETM Express Enzyme (from Life Technologies Japan Ltd.), TrypLETM Select Enzyme (from Life Technologies Japan Ltd.), DISPASE (registered trademark), and the like can be utilized. For example, in cases where trypsin is used for dissociation, the lower limit of the concentration of trypsin in a solution is not particularly limited as far as it may disperse the cell spheroids, and can be, for example, 0.15 vol% or more, 0.18 vol% or more, 0.20 vol% or more, or 0.24 vol% or more. On the other hand, the upper limit of the concentration of trypsin in a solution is not particularly limited as far as there is no influence such as the dissolution of the cells themselves, and may be 0.30 vol% or less, 0.28 vol% or less, or 0.25 vol% or less. In addition, the time for treatment depends on the concentration of trypsin, but the lower limit of the length is not particularly limited as far as the cell spheroids may be dispersed sufficiently by the action of trypsin, and may be, for example, 5 minutes or more, 8 minutes or more, 10 minutes or more, 12 minutes or more, or 15 minutes or more. On the other hand, the upper limit of the length for treatment is not particularly limited as far as there is no influence, for example, the dissolution of the cells themselves by the action of trypsin, and may be, for example, 30 minutes or less, 28 minutes or less, 25 minutes or less, 22 minutes or less, 20 minutes or less, or 18 minutes or less. In this regard, in cases where a commercially available releasing agent is used, the releasing agent can be used at a concentration at which the cells can be dispersed to a single state, as stated in the protocol attached thereto. Treatment with the releasing agent and/or chelator, followed by light physical treatment, can promote dissociation. This physical treatment is not limited, but examples of such a treatment includes a method in which a solution containing the cells is pipetted a plurality of times. Furthermore, the cells may be allowed to pass a strainer or a mesh, if necessary.

The dissociated cells can be collected after being left to stand or centrifuged and removing the supernatant containing a releasing agent. The collected cells may be washed, if necessary.

The period for the stabilization culture is not particularly limited, and is preferably 4 hours or more in terms of the high sphericity and narrow spheroid diameter distribution of the cardiomyocyte spheroids. The period for the stabilization culture is more preferably 12 hours or more, still more preferably 24 hours or more, particularly preferably 48 hours or more, most preferably 60 hours or more. In addition, the period for the stabilization culture is preferably 1000 hours or less in terms of economic efficiency, more preferably 500 hours or less, still more preferably 240 hours or less, particularly preferably 150 hours or less, most preferably 84 hours or less. During the period for stabilization culture, the composition of the liquid (culture medium) component may be changed. The liquid can usually be replaced by an operation called culture medium replacement in the art. The frequency of replacement is not particularly limited, and may be performed regularly, for example, every 1 hour, every 4 hours, every 8 hours, every 12 hours, every 24 hours, every 48 hours, or randomly. In addition, the amount of replacement is also not limited, and may be almost the whole amount or the half amount, or predetermined amount may be continuously replaced through perfusion.

In the present invention, the time taken to produce cardiomyocyte spheroids during which dissociated cardiomyocytes are aggregated under suspension conditions is not particularly limited, but is preferably within 216 hours in terms of providing such a cell spheroids for cardiomyocytes transplant treatment or the like in a short time, more preferably within 132 hours, still more preferably within 108 hours, particularly preferably within 84 hours, most preferably within 60 hours. In addition, the time taken to produce the cardiomyocyte spheroids is preferably 25 hours or more, particularly preferably 36 hours or more, most preferably 48 hours or more, in terms of increasing the possibility to obtain cardiomyocyte spheroids that beats properly.

In the present invention, the temperature at which dissociated cardiomyocytes are aggregated under suspension conditions to produce cardiomyocyte spheroids is not particularly limited, but is preferably equal to or higher than a temperature at which neither the cells nor the suspension is frozen, and equal to or lower than the boiling point of the suspension. The temperature is preferably 4°C or more, more preferably 10°C or more, still more preferably 18°C or more, particularly preferably 25°C or more, most preferably 32°C or more, in terms of increasing the possibility to start beating in a short time. In addition, the temperature is preferably 60°C or less, more preferably 50°C or less, still more preferably 45°C or less, particularly preferably 43°C or less, most preferably 40°C or less, in terms of reducing the possibility to cause cell death.

In the present invention, the composition and concentration of the gas in an environment in which dissociated cardiomyocytes are aggregated under suspension conditions to produce cardiomyocyte spheroids are not particularly limited, and the production can be performed in an incubator under a carbon dioxide gas atmosphere as commonly used. The concentration of the carbon dioxide gas in this case is also not particularly limited. The concentration of 1% or more and 10% or less may retain the liquid property of the suspension. The concentration range of the carbon dioxide gas is more preferably 2% or more and 8% or less, still more preferably 3% or more and 7% or less, particularly preferably 4% or more and 6% or less, most preferably 5%. In addition, the concentration of the oxygen is also not particularly limited, and is preferably 5% or more in terms of reducing the possibility to cause cell death, more preferably 10% or more, still more preferably 15% or more, particularly preferably 18% or more, most preferably 20% or more. In addition, the concentration is preferably 30% or less in terms of inhibiting unnecessary oxidation of the cells, and is more preferably 27% or less, still more preferably 25% or less, particularly preferably 22% or less, most preferably 21% or less.

In the present invention, the pH of a suspension in which dissociated cardiomyocytes are aggregated under suspension conditions to produce cardiomyocyte spheroids is not particularly limited. The pH is preferably 2 or more in terms of reducing the possibility of being influenced by unnecessary oxidation, and the pH is preferably 12 or less in terms of reducing the possibility to cause cell death. The range of the pH is more preferably 3 or more and 11 or less, still more preferably 4 or more and 10 or less, particularly preferably 5 or more and 9 or less, most preferably 6 or more and 8 or less.

In the present invention, the amount of dissolved oxygen in the suspension in which dissociated cardiomyocytes are aggregated under suspension conditions to produce cardiomyocyte spheroids is not particularly limited, but is preferably 10 or more in terms of inhibiting cell death, the amount is preferably 100 or less in terms of eliminating the necessity of extra aeration, and the amount is more preferably 25 or more and 95 or less, still more preferably 40 or more and 90 or less, particularly preferably 50 or more and 85 or less, most preferably 60 or more and 80 or less, assuming that the amount of the dissolved oxygen when the liquid component of the suspension is saturated with a sufficient amount of air is 100.

In the present invention, the component in the liquid to be used for suspension when dissociated cardiomyocytes are aggregated under suspension conditions to produce cardiomyocyte spheroids is not particularly limited, but it is preferable to use culture medium that enables cardiomyocytes to undergo stabilization culture. In addition, since, according to the present invention, a favorable cardiomyocyte spheroids can be obtained in a simple manner, it is preferable, in terms of economic efficiency, a side effect during treatment, and the easiness of post-treatment, that no special culture medium component is used for 26 hours or more, be, more preferably for 24 hours or more, still more preferably 12 hours or more, particularly preferably 6 hours or more when dissociated cardiomyocytes are aggregated to produce cardiomyocyte spheroids. Most preferably, such a component is never used. Examples of special culture medium component include insulin, transferrin, selenium, basic fibroblast growth factor (bFGF), epidermal growth factor (EGF), platelet-derived growth factor-BB (PDGF-BB), endothelin-1 (ET-1), and the like. The liquid for suspension can usually be replaced by an operation called culture medium replacement in the art. The frequency of replacement is not particularly limited, and may be performed periodically, for example, every 1 hour, every 4 hours, every 8 hours, every 12 hours, every 24 hours, every 48 hours, or randomly. In addition, the amount of replacement is also not limited, and may be an almost whole amount or the half amount, or predetermined amount may be continuously replaced through perfusion.

The method for producing according to the present invention allows to obtain cardiomyocyte spheroids having a favorable shape and high sphericity. Examples of an indicator of shape include an aspect ratio. The aspect ratio can be determined by the ratio between the minimum Feret diameter and the maximum Feret diameter. In a preferable aspect of cardiomyocyte spheroids that can be provided in the present invention, the average aspect ratio is 0.70 or more, more preferably 0.75 or more, still more preferably 0.80 or more, particularly preferably 0.85 or more, most preferably 0.90 or more.

In addition, the method for producing according to the present invention allows to provide cardiomyocyte spheroids population having a relatively homogeneous size. Examples of an indicator of homogeneity of size include a standard deviation. In a preferable aspect of cardiomyocyte spheroids that can be provided by the present invention, the standard deviation of the size of the cell spheroids is 30% or less, still more preferably 25% or less, particularly preferably 20% or less, most preferably 15% or less based on the average size. The average size of the cardiomyocyte spheroids is not particularly limited, and is preferably 50 µm or more in terms of expressing the functions as cardiomyocytes (beating and the like), more preferably 75 µm or more, still more preferably 100 µm or more, particularly preferably 150 µm or more, most preferably 200 µm or more. In addition, the average size of the cardiomyocyte spheroids is preferably 2000 µm or less in terms of easiness to inject the cells during transplant, more preferably 1500 µm or less, still more preferably 1000 µm or less, particularly preferably 500 µm or less, most preferably 300 µm or less. As used herein, the size of the cell spheroids is determined by the average of the minimum Feret diameter and the maximum Feret diameter.

Cardiomyocyte spheroids obtained in accordance with the present invention can be transplanted into the heart tissue of an individual (organism). For example, cardiomyocytes can be directly injected into the heart tissue. In cases where cardiomyocyte spheroids are produced without a special liquid component, the cardiomyocyte spheroids can be directly used for transplant without replacing the liquid component for suspension, but may also be transplanted into an individual after the liquid component is replaced with a suitable liquid (for example, physiological saline). Cardiomyocyte spheroids obtained in accordance with the present invention has a narrow size distribution and a favorable shape, and thus, hardly poses a drawback such as the clogging inside an injection needle, but just in case, it is preferable to preliminarily collect only cardiomyocyte spheroids that have been able to pass through an injection needle, a flow path, or a sieve with an inner diameter equal to or smaller than an injection needle to be used for transplant. In cases where it is intended to use a low invasive injection needle of 30 G or less in particular, it is effective to remove cardiomyocyte spheroids that have not passed an injection needle in advance. In addition, the size of a cell spheroid represented by the number of cells that constitute the spheroids is preferably 100 or more in terms of increasing the ratio of engraftment for the heart, more preferably 500 or more, still more preferably 750 or more, particularly preferably 900 or more, most preferably 1000 or more. The size is preferably 5000 or less in terms of reducing the variation in the quality of the cells in the cell spheroid, and easiness to perform a transplant using an injection needle, more preferably 4000 or less, still more preferably 3000 or less, particularly preferably 2000 or less, most preferably 1500 or less.

As described above, there is no other known example in which a great number of preferable cardiomyocyte spheroids can be obtained at one time in a simple manner as in the present invention and thus, the present invention is a very excellent epoch-making invention.

### Examples

The present invention will now be described with reference to Examples, but the present invention should not be limited to these Examples.

The materials and ingredients used are listed in Table 1.

**[Table 1]**

| Classification | Product Name | Manufacturer | Model Number |
|---|---|---|---|
| Cardiomyocytes | CarmyA-Human | Myoridge | H-011106 |
| Seeding Culture Medium | Seeding Culture Medium for CarmyA | Myoridge | ME-02 |
| Maintenance Culture Medium | Maintenance Culture Medium for CarmyA | Myoridge | ME-01 |
| Additive | Y-27632 | Wako | 036-24023 |
| Culture Container | 24-well plate | Coming | 3524 |
| | 12-well plate | Sumitomo Bakelite Co., Ltd. | MS8012R |
| | Cell culture plate 96-well, Round Bottom | WATSON | 197-96UCS |
| Coating Agent | Gelatin, from bovine bone | Wako | 071-06291 |
| RNA Eluent | Trizol Reagent | ThermoFisher | 15596026 |
| Buffer | PBS | gibco | 10010-031 |
| Releasing Agent | Accumax | Inovative Cell Technologies, INC | AM105 |

According to the documents attached to the cardiomyocytes used, the cardiomyocytes were frozen cells, and were human cardiomyocytes derived from iPS cells. In addition, the cell line of the iPS cell was 253G1. The ratio of cardiac troponin T-positive cells was 95.5%. The basic composition of maintenance culture medium for CarmyA was stated as follows: CaCl₂, 0.182 g/L; MgSO₄, 0.09767 g/L; KCl, 0.439 g/L; NaHCO₃, 3.362 g/L; NaCl, 5.4525 g/L; Na₂HPO₄, 0.109 g/L; HEPES, 5.958 g/L; Phonol red Na, 0.0134 g/L. Y-27632 in Table 1 is a ROCK inhibitor.

### (Preparation of Gelatin Solution)

100 mg of gelatin was dissolved in 100 mL of pure water (prepared with Milli-Q manufactured by Merck & Co., Inc.), and the resulting solution was autoclaved and stored at 4°C.

### (Thawing of Cells)

1) 500 µL of the gelatin solution was added to one well in a 24-well plate, and incubated at 37°C for 1 hour.
2) Thawing culture medium, which is seeding culture medium for CarmyA, supplemented with Y-27632 at a final concentration of 10 µM, was warmed up to 37°C.
3) Two vials containing freeze-preserved CarmyA-Human were warmed up in a water bath at 37°C for 3 minutes.
4) Without pipetting, 50 mL of the cell suspension was transferred into each centrifuge tube.
5) The vials were washed with 1 mL of the thawing culture medium, 1 mL of the resulting medium was added to and mixed with the cell suspension at a speed of 1 drop/5 seconds and with shaking.
6) 1 mL of the thawing culture medium was added to and mixed with the cell suspension at 1 drop/2 seconds and with shaking.
7) 8 mL of the thawing culture medium was added to and mixed with the cell suspension at 1 drop/1 second and with shaking.
8) The resulting liquid was centrifuged at 300× g for 5 minutes, and then, the supernatant was removed.
9) The resulting liquid was suspended in 500 µL of the thawing culture medium, and then, the resulting suspension in 2 tubes was put together. The number of cells was counted using a Countess II FL automatic cell counter manufactured by Thermo Fisher Scientific Inc.
10) With reference to the results of the count of cells, 500 µL of the 2 × 10⁶ cells/mL cell suspension and 5 mL of the 2 × 10⁵ cells/mL cell suspension were prepared using the thawing culture medium.

### (Production of Cardiomyocyte Spheroids - without Stabilization Culture)

The 2 ×10⁵ cells/mL cell suspension and the thawing culture medium were added to each container according to the number of cells seeded stated in Table 2. The containers were lidded. An Orbital Shaker OS-762 (that is rotated along the horizontal plane in a circle with a shaking width (diameter) of 25 mm) manufactured by Optima Inc. was placed under 5% CO₂ at 37°C in an incubator. A container containing cardiomyocytes suspension was placed on a stage in this shaker. The stage was rotated at a predetermined rotational speed to start the production of cardiomyocyte spheroids. After 48 hours, all the supernatant was removed. All the culture medium was replaced with a maintenance culture medium at 37°C, supplemented with Y-27632 at a final concentration of 5 µM. The resulting suspension continued to be rotated until 72 hours later. The results are shown in Table 2. The cells and the cell spheroids having an average size of 50 µm or more after 72 hours were regarded that a cell spheroid had been formed. The size of each of the cells and cell spheroids was calculated as the average of both the minimum Feret diameter and the maximum Feret diameter that were determined from the images of the cells and cell spheroids photographed under a routine inverted microscope Primovert manufactured by Carl Zeiss AG, using the measuring function of a software article ZEN attached to the microscope. As the average size of the cell spheroids, the average size of the cell spheroids with the size of 50 µm or more was used. The standard deviation of the size of the cell spheroids was calculated as the positive square root of the square mean of a difference between the average and each value, which was used to determine the average size of the cell spheroids. As the aspect ratio, the ratio between the minimum Feret diameter and the maximum Feret diameter was used. The beating verification time is the duration from the start of the production of the cardiomyocyte spheroids to the time when beating was verified. In Examples 5 and 6, approximately 30% of all the cells were dead cells, suggesting that the rotation was a little strong.

**[Table 2]**

| | Rotation Rate [rpm] | Number of Cells Seeded/Total Amount of solution | Container | Formation of Cell Spheroid | Cell Spheroid Beating Verification Time | Average Size of Cardiomyocyt e Spheroid [µm] | Average Aspect Ratio | Standard Deviation of Size of Cell Spheroid |
|---|---|---|---|---|---|---|---|---|
| Comparative Example 1 | 0 | 1×10⁴ cells/100 µL | Round-bottom 96-well | Not Formed | - | - | - | - |
| Comparative Example 2 | 0 | 5×10³ cells/100 µL | Round-bottom 96-well | Not Formed | - | - | - | - |
| Comparative Example 3 | 0 | 2.5×10³ cells/100 µL | Round-bottom 96-well | Not Formed | - | - | - | - |
| Comparative Example 4 | 0 | 1×10³ cells/100 µL | Round-bottom 96-well | Not Formed | - | - | - | - |
| Example 1 | 83 | 1×10⁵ cells/1 mL | Flat-bottom 12-well | Formed | 48 hours | 735 | 0.70 | - |
| Example 2 | 83 | 2×10⁵ cells/1 mL | Flat-bottom 12-well | Formed | 48 hours | 1224 | 0.33 | - |
| Example 3 | 100 | 1×10⁵ cells/1 mL | Flat-bottom 12-well | Formed | 48 hours | 527 | 0.99 | - |
| Example 4 | 100 | 2×10⁵ cells/1 mL | Flat-bottom 12-well | Formed | 48 hours | 874 | 0.90 | - |
| Example 5 | 120 | 1×10⁵ cells/1 mL | Flat-bottom 12-well | Formed | 48 hours | 97 | 0.79 | 21 |
| Example 6 | 120 | 2×10⁵ cells/1 mL | Flat-bottom 12-well | Formed | 48 hours | 140 | 0.76 | 27 |

### (Production of Cardiomyocyte Spheroids - with Stabilization Culture)

1) A gelatin solution was added to each container up to the recommended volume for the container, and left to stand at 37°C for 1 hour, and then the gelatin solution was removed.
2) 500 µL of the cell suspension prepared at 2 × 10⁶ cells/mL in the paragraph (Thawing of Cell) was seeded on the gelatin-coated well.
3) To eliminate unevenness in the adhesion of the cells, the suspension was left to stand in a clean bench for 15 minutes and then transferred into 5% CO₂ at 37°C in an incubator.
4) The stabilization culture was continued for 72 hours. All the supernatant was removed and all the culture medium was replaced with a maintenance culture medium at 37°C every 24 hours.
5) The supernatant was removed by aspirator, and then, and 1 mL of PBS was added.
6) The PBS was removed, and then, 0.25 mL of Accumax was added, and the resulting suspension was incubated at 37°C for 20 minutes.
7) 1 mL of seeding culture medium (containing 3 µM Y-27632) was added, and the resulting suspension was left to stand for 5 minutes.
8) The suspension was pipetted until the cell spheroids were no longer visually observable, and then, transferred into a 15 mL centrifuge tube.
9) The suspension was centrifuged at 300× g for 5 minutes, and then, the supernatant was removed.
10) 1 mL of seeding culture medium (containing 10 µM Y-27632) was added, and the number of cells was counted.
11) Based on the results of counting, 2.5 mL of the 2 × 10⁵ cells/mL cell suspension was prepared.
12) The 2 ×10⁵ cells/mL cell suspension produced in 11) and the thawing culture medium were added to each container according to the number of cells seeded stated in Table 3. The containers were lidded.
13) An Orbital Shaker OS-762 (with a rotation range of 25 mm) manufactured by Optima Inc. was placed under 5% CO₂ at 37°C in an incubator. A container containing cardiomyocytes suspension was placed on a stage in this shaker. The stage was rotated at a predetermined rotational speed to start the production of cardiomyocyte spheroids.
14) After 24 hours, all the supernatant was removed, and all the culture medium was replaced with maintenance culture medium supplemented with Y-27632 at a final concentration of 5 µM at 37°C.
15) After 48 hours, all the supernatant was removed and all the culture medium was replaced with maintenance culture medium supplemented with Y-27632 at a final concentration of 2 µM at 37°C.
16) The rotation was continued until 72 hours later. The results are shown in Table 3. The cells and the cell spheroids having an average size of 50 µm or more after 72 hours were regarded that cell spheroids had been formed. The beating verification time is the length of time from the start of the production of the cardiomyocyte spheroids to the time when beating was verified. In Example 7, the same operation as in Example 4 was performed except that stabilization culture was performed. The average size of the cell spheroid was enabled to be smaller than in Example 4, and the ratio of dead cells was approximately 2% of all the cells.

**[Table 3]**

| | Rotation Rate [rpm] | Number of Cells Seeded/Total Amount of Solution | Container | Formation of Cell Spheroid | Cell Spheroid Beating Verification Time | Average Size of Cardiomyoc yte Spheroid [µm] | Average Aspect Ratio | Standard Deviation of Size of Cell Spheroid |
|---|---|---|---|---|---|---|---|---|
| Comparative Example 5 | 0 | 1×10⁴ cells/100 µL | Roundbottomed 96-well | Not Formed | - | - | - | - |
| Comparative Example 6 | 0 | 5×10³ cells/100 µL | Roundbottomed 96-well | Not Formed | - | - | - | - |
| Comparative Example 7 | 0 | 2.5×10³ cells/100 µL | Roundbottomed 96-well | Not Formed | - | - | - | - |
| Comparative Example 8 | 0 | 1×10³ cells/100 µL | Roundbottomed 96-well | Not Formed | - | - | - | - |
| Example 7 | 100 | 2×10⁵ cells/1 mL | Flat-bottomed 12-well | Formed | 48 | 256 | 0.91 | 36 |

The results in Table 2 and Table 3 have revealed that the present invention can provide cardiomyocyte spheroids by an extremely simple method. In addition, the present invention allows to start beating in an extremely short time, compared with a conventional method for producing cardiomyocyte spheroids. In addition, it was revealed that the present invention allows to control the size of cardiomyocyte spheroids freely. In addition, it was revealed that the shape and size distribution of the cardiomyocyte spheroids are favorable. These Examples are only a part of the present invention, but sufficiently demonstrate that the present invention allows to produce a large amount of cardiomyocyte spheroids in an extremely simple manner in a short time. Thus, the present invention is expected to have a significant contribution to a transplant treatment or the like.

All the publications, patents, and patent applications cited herein are incorporated herein by reference in their entirety.

## Claims

1. A method for producing cardiomyocyte spheroids, comprising an aggregation step of allowing dissociated cardiomyocytes having a history of stabilization culture to flow under suspension conditions to aggregate the cells.

2. A method for producing cardiomyocyte spheroids, comprising an aggregation step of allowing dissociated cardiomyocytes to flow under suspension conditions in a non-annular container to aggregate the cells.

3. The method for producing cardiomyocyte spheroids according to claim 1 or 2, wherein the stabilization culture is adherent culture.

4. The method for producing cardiomyocyte spheroids according to any one of claims 1 to 3, wherein the period for the stabilization culture is 4 hours or more.

5. The method for producing cardiomyocyte spheroids according to any one of claims 1 to 4, wherein the time taken to produce cardiomyocyte spheroids from the dissociated cardiomyocytes is within 216 hours.

6. The method for producing cardiomyocyte spheroids according to any one of claims 1 to 5, wherein the container is substantially flat-bottomed.

7. The method for producing cardiomyocyte spheroids according to any one of claims 1 to 6, wherein the container has a capacity of 0.5 mL or more.

8. The method for producing cardiomyocyte spheroids according to any one of claims 1 to 7, wherein the flow is performed at a rotation speed of 20 rpm or more and less than 200 rpm.

9. The method for producing cardiomyocyte spheroids according to claim 8, wherein the container is rotated in the range of 10 mm or more.

10. The method for producing cardiomyocyte spheroids according to any one of claims 1 to 9, wherein, as a liquid to be used for the suspension, culture medium containing one or more components selected from the group consisting of insulin, transferrin, selenium, basic fibroblast growth factor (bFGF), epidermal growth factor (EGF), platelet-derived growth factor-BB (PDGF-BB), and endothelin-1 (ET-1) is not used for 26 hours or more.

11. The method for producing cardiomyocyte spheroids according to any one of claims 1 to 10, wherein the cardiomyocytes are derived from a pluripotent stem cell(s).

12. Cardiomyocyte spheroids produced by the method according to any one of claims 1 to 11 and having an average aspect ratio of 0.70 or more.

13. Cardiomyocyte spheroids produced by the method according to any one of claims 1 to 11, wherein the standard deviation of the size of cardiomyocyte spheroids is 30% or less of the average size thereof.

14. Cardiomyocyte spheroids produced by the method according to any one of claims 1 to 11, wherein the average size of cardiomyocyte spheroids is 50 µm or more and 2000 µm or less.

15. Cardiomyocyte spheroids produced by the method according to any one of claims 1 to 11, wherein cardiomyocyte spheroids start beating within 216 hours from the start of production of cardiomyocyte spheroids.
